# EUROPEAN PATENT SPECIFICATION

(11) **EP 1 610 652 B1**
(45) Date of publication and mention of the grant of the patent: **14.05.2008**
(21) Application number: 02808200.6
(22) Date of filing: 04.12.2002
(51) Int. Cl.: A47F 1/035, B01F 15/04, B01F 9/02

(54) **A MACHINERY & PROCESS TO MANUFACTURE INSTANTLY A BATCH OF CUSTOMISED DOSAGE**
VORRICHTUNG UND VERFAHREN ZUR SOFORTIGEN HERSTELLUNG EINER INDIVIDUELLEN DOSIERUNGSCHARGE
MACHINES ET PROCEDE DE FABRICATION INSTANTANEE D'UN LOT A POSOLOGIE PERSONNALISEE

(43) Date of publication of application: 04.01.2006
(73) Proprietor: Kamineni, Shobana, Andhra Pradesh (IN)
(72) Inventor: Kamineni, Shobana, Andhra Pradesh (IN)
(74) Representative: Chaillot, Geneviève
(86) International application number: PCT/IN2002/000229
(87) International publication number: WO 2004/049877

(56) References cited:
- WO-A1-98/25695
- FR-A- 2 779 664
- US-A- 4 372 686
- US-A- 5 690 283
- US-A- 6 057 514
- 1993, BERLIN: ULLSTEIN MOSBY, ISBN 3-86126-013-1 article VOIGHT R.: 'Pharmazeutische technologie für studium und beruf', pages 224 - 225, XP008056334 chapter 9.4.2.2.

## Description

The present invention is the machinery and process of manufacture of pharmaceutical formulations instantly needed by the consumer, depending on the dosage required by the individuals.

More particularly, based on a computer rule engine programe, the required dosage is estimated. This is communicated through logic controllers, and the invention relates to an instant process where in different inputs of ingredients are put from the different hoppers on the top, and the ingredients are taken according to the ratio and weight measurement required. The whole content is sent down through as tube, which passes through as mixer/blender, to the dosage-measuring unit and to dosage conversion unit where the whole mixture depending upon the dosage & size the tablets/capsules/sachets are formed and delivered to the consumer.

The production of large-scale pharmaceuticals is a process that has been known for a long time. The known processes, however involve notable shortcomings, and heretofore none of the known processes has been successfully used in manufacturing instant formulations to the need of the consumer.

The shortcomings of the prior art processes include; the large dosage of the pharmaceutical medicines to all, depending upon the availability from the large-scale manufacturers.

The same dosage for all individuals, mostly differentiating between children & adults, but not between age, weight, sex, lifestyle etc.,

The time scale for production of such pharmaceuticals is very lengthy and on a high scale industrial process.

The non-availability of some medicines at times involves the breakdown of any one machine in the whole process.

The non-availability of a combination of different medicines in one form, as a result of which the consumer has to take more forms of tablets/capsules/or any other form, which may affect him Psychologically, and physiologically.

Medical professionals have to depend on the choice of the large-scale industrial sector rather depending on a prescription directed to the need of the consumer to act upon a particular disease of an individual.

All of the above and other unfavorable factors have made it necessary for the consumer, to have the machinery and process invented hereto for carrying out the manufacture instantly of a batch of customized dosage.

Types of blending machine are described in US-A-5690283, FR 2779664 & US-4-372686

US 5 690283 discloses a blending machinery for blending two or more different types of coffee beans, comprising a plurality of supply hoppers, a scale at the exit of the hoppers, and two units, one bagging units and one grinding unit.

FR 2 779 664 discloses an machinery for mixing seeds comprising a plurality of hoppers, whose discharge openings are controlled automatically through the use of a computer. The discharge opening opens directly on a mixing plate. The seeds, once mixed on the plate, are then discharged through an opening in the plate into a container.

It is therefore the primary object of the present invention to make available to the needy or consumer, instantly, upon requirement and based upon age, weight, sex, disease, lifestyle etc., along with the exact requirement of generics and dosage, quantities for each individual.

Here the invented process of manufacture is carried out through machinery invented to manufacture instantly, which runs on a separately made programe (rule engine) of the computer. The invented machine has the technical features disclosed in claim 1.

In the process of manufacture of customized dosage there are seven phases of the process in a single machine.
1. Generation of Customized Formula and Control
2. Measurements and Drawing of Ingredients
3. Homogeneous Mixing of Ingredients
4. Measurement of Dosage
5. Dosage Conversion
6. Automatic Cleaning
7. Dispensing

In the first phase the Computer receives inputs, which also Controls all process plant operations through the Microprocessor based Control Unit. Based on individual requirements and certain criteria, Rule Engine Software module generates customized dosage parameters like ingredients, dosage and duration.

Based on this information computer selects the bins, quantities to be drawn from each bin. It also determines the process parameters for manufacture. These Parameters are found and set earlier by predetermined experimental methods.

In the second phase, it has a multi pack assembly. Individual ingredient pack with open / close mechanism can be replaced as and when required. Measure And Draw Mechanism is fully controllable by the Rotary mechanism. Sensors aid accurate measurement and drawing of required ingredients from various packs. The actual value of the measure is determined by the position of the Piston in a Cylinder. The positioning of the piston is controlled. This position versus unit measure is pre calibrated and stored. This calibration is unique for a given characteristic of the ingredient. All such measured and drawn ingredients are delivered into a common container through independent conveying chutes / tubes.

All the ingredients that pass through this container are delivered directly into the blender through a common Interface Unit.

Interface Unit is basically a conduit tube connecting the Measure And Draw Mechanism and the Blending Unit. One end of the common Interface Unit is permanently attached to the common collector and the other end carries a controllable Engagement Mechanism that is used to Attach / Detach from the Blending Unit. This controllable Engagement Mechanism links the common Interface Unit with the Blending Unit whenever the Measure and Draw Mechanism is in operation to ensure free and spill free deliver of the ingredients into the Blending Unit. Thus, at the end of all the operations of the Measure and Draw Mechanism, the blender will contain the required amount of each of the ingredients as determined by the computer.

The blender contains inner blades for proper blending. The rotations of the blender are controlled through a motor and Support arm.

In the third phase, it has comprehensive computer controlled mixing mechanism to provide various blending cycles. It can control various parameters like number of cycles, angles of rotation, speed of rotation. Blending cycle programs are generated in advance, based on certain criteria.

The mixing concept of the Blending Unit is by revolving a double conical container about a symmetrical radial axis. This unit contains a fixed internal changeable Kneading Unit that effectively and uniformly mixes the ingredients as the container revolves about the axis.

This unit also has a controllable opening and closing mechanism at both apex ends of the conical sections. This mechanism allows free entry for ingredients while drawing them into the Blending Unit and closing the unit while under operation. Also, the apex conical end is adopted for easy engagement / disengagement with the adjacent units.

After the Measure and Draw Mechanism operation is completed, the top conical end is disengaged with the common Interface Unit and closed. These operations ensure that the Blending Unit is independent and isolated from other units and is ready for its operation. Blender operates as per predetermined blending cycle.

Once the mixture cycle is completed and the Blending Unit has stopped in the predetermined position, the controllable Engagement Mechanism at the bottom end engages with the receiving chute of the Mixture Storage Unit. After this engagement, the bottom end of the conical section opens up allowing the homogeneously mixed ingredients to flow past it.

In the fourth phase unit consists of two main sub units namely the Mixture Storage Unit and the Dosage Measuring Unit.

The main function of the Mixture Storage Unit is to store the homogeneous mixture and feed to the Dosage Measuring Unit for the purpose of dosage.

This unit has a controllable Engagement Mechanism at the top of the chute attached to the cover of this unit. The bottom of the chute also has a mechanism used during the cleaning operations. The operation of these mechanisms is fully controllable.

Dosage Measuring Unit is one of the main units in the whole assembly and is critical in nature. The volumetric measuring works on the principle of a square piston and a square box, where the position of the square piston determines the volume to be measured. The volumetric space is the space enclosed by the frame on 3 sides, square piston on one side, one sluice gate at the mixture entry side and one sluice gate at the delivery side. The computer controls the operations of this sluice gate.

The positioning of the piston is pre calibrated to achieve the required volumetric space and is unique for the given mixture ingredients. The pre calibration is carried out by experimental methods to ensure the correct dosage.

In the dosage feeding operation, sluice gates are opened and closed in a sequence to deliver the dosage.

In the fifth phase unit contains Compression Unit / Capsule filling / Sachet filling modules. Measured Dosage is transferred through a chute to a predefined place.

Compression Unit consists of a rotary table with indexing facility to stop, fill, punch and eject. Dosage transfer takes place when the table is at halt position. Material does not spill and hence no wastage. Formation of a tablet is by compression using a rotating cam mechanism.

Capsule filling module consists of separation of the cap and body of the empty hard gelatin capsule, filling and capping mechanism.

Sachet filling module contains form, fill, and seal mechanism. Computer controls and synchronizes all these operations. In the sixth phase material-handling units like Chutes, Blender, Dosage feeding Mechanism and Conversion Unit are cleaned automatically by vacuum cum pressure method. The particulate matter is collected in a disposable bin.

In the seventh phase the following results shall obtain with particular combinations of medicines/pharmaceutical formulations, with particular dosages in the form of tablets / capsules / sachets which fall in a container. Computer prints a sticker label. It will have details like date, Name of Person, Product name, composition, dosage per day, duration for consumption and other information. Sticker is pasted on the container and is dispensed.

With reference to the aforesaid,

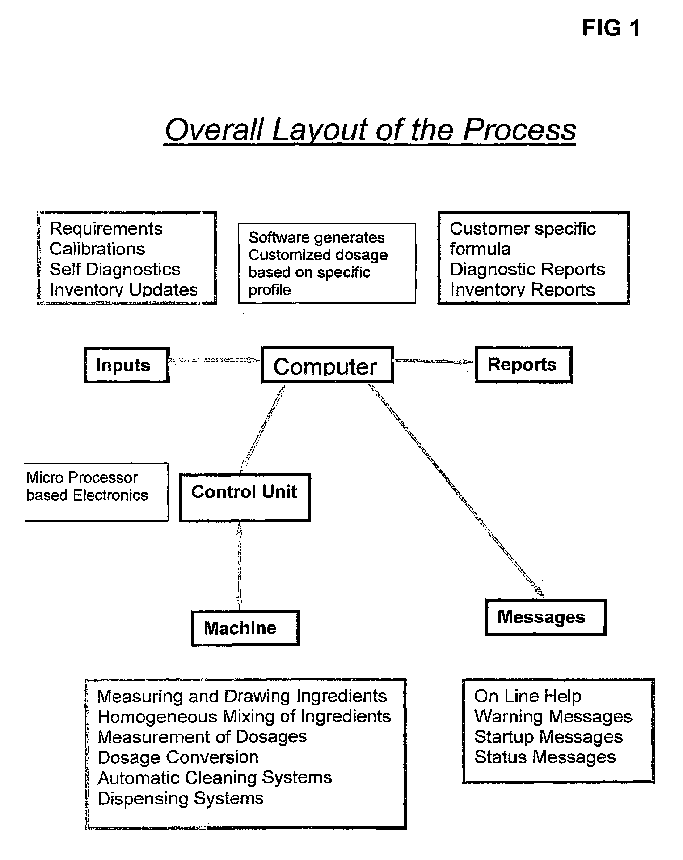

**FIG. 1** is the drawing shows the overall layout of the process through as flow chart.

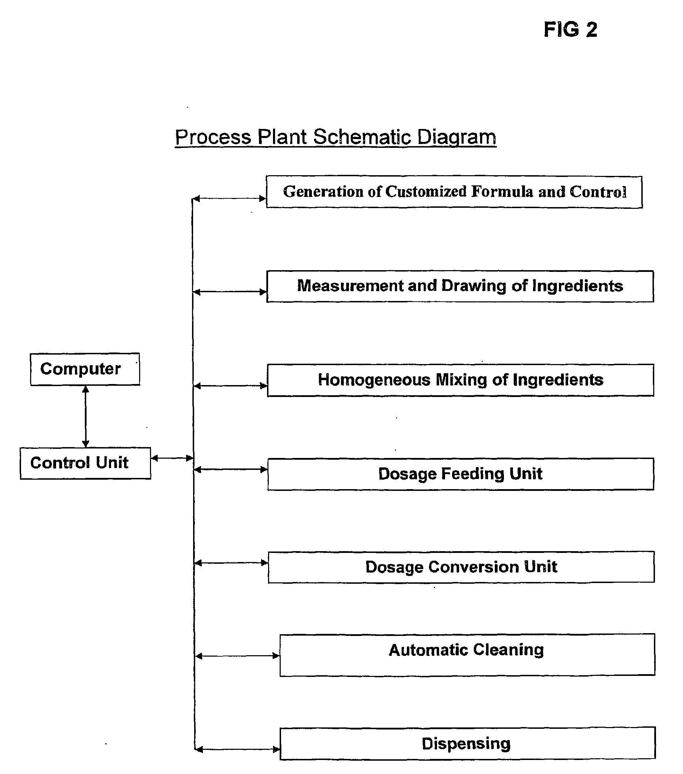

**FIG. 2** is the process plant schematic diagram

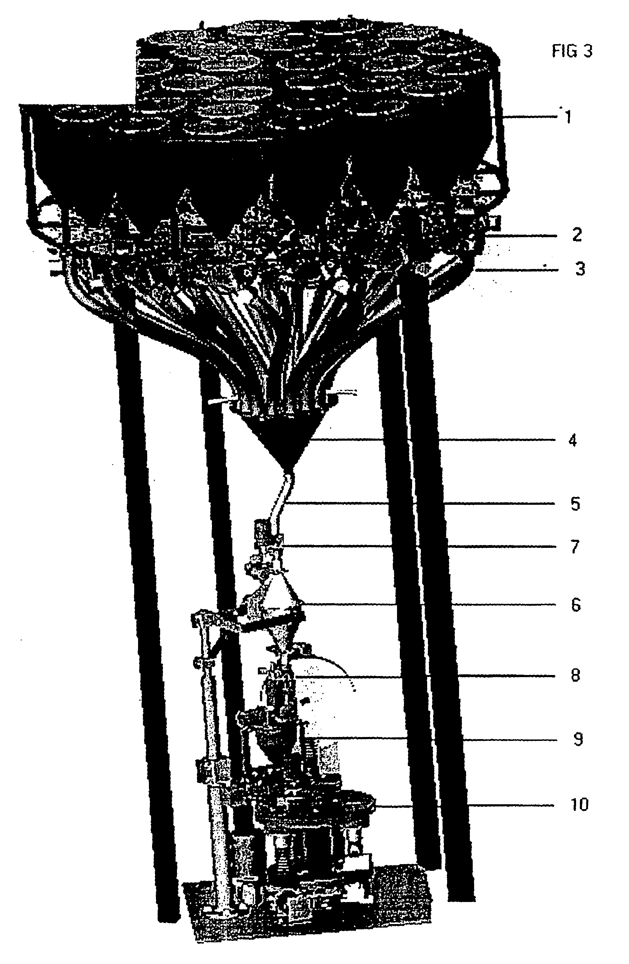

**FIG. 3** is the view of the machinery in the lines of complete process of manufacture.

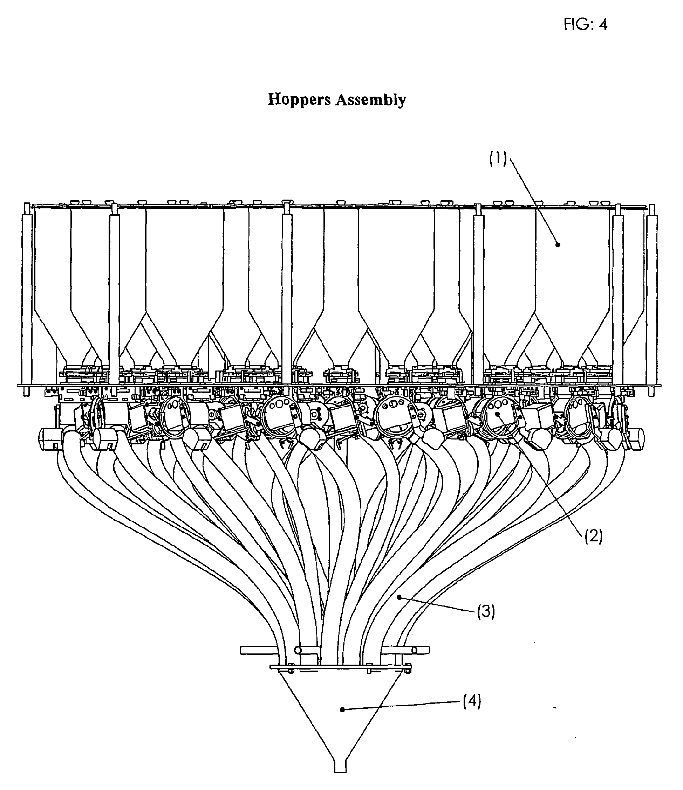

**FIG. 4** is the diagram showing the Hoppers assembly unit of the invented machinery.

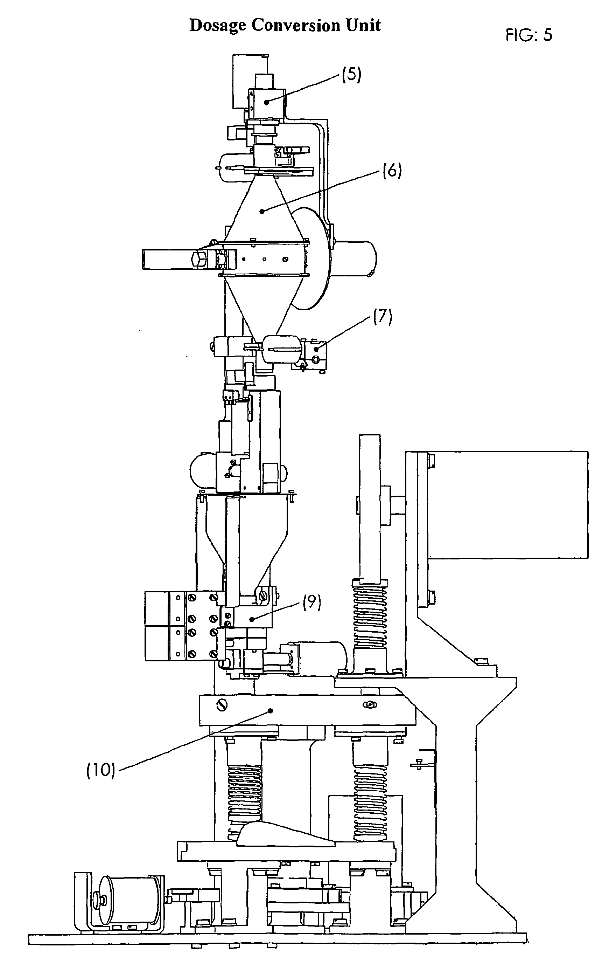

**FIG. 5** is the diagram showing the Dosage Conversion unit of the invented machinery.

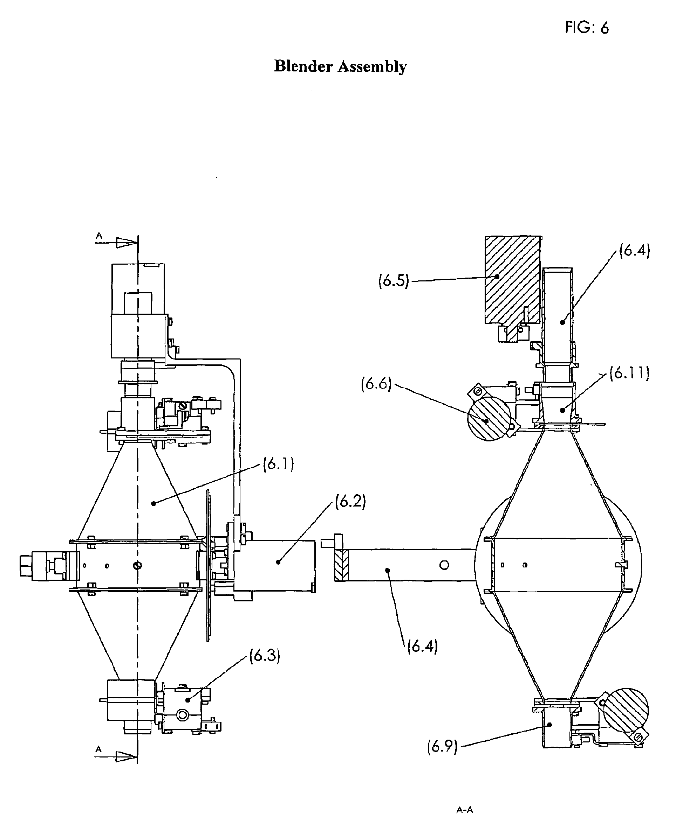

**FIG. 6** is the diagram showing the Blender Assembly unit of the invented machinery.

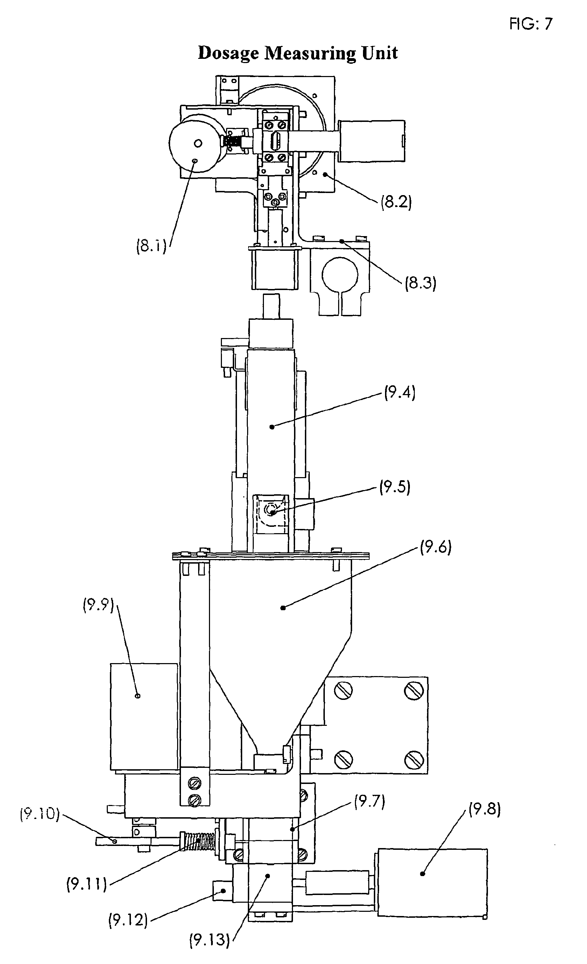

**FIG. 7** is the diagram showing the Dosage measuring unit of the invented machinery.

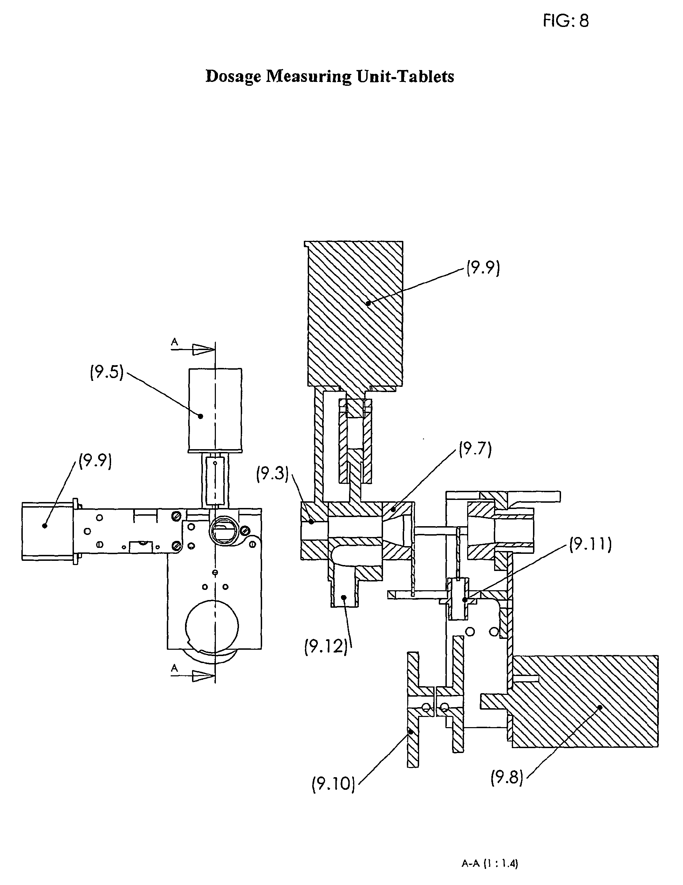

**FIG. 8** is the diagram showing the Dosage measuring unit in particular to the tablet form of the invented machinery.

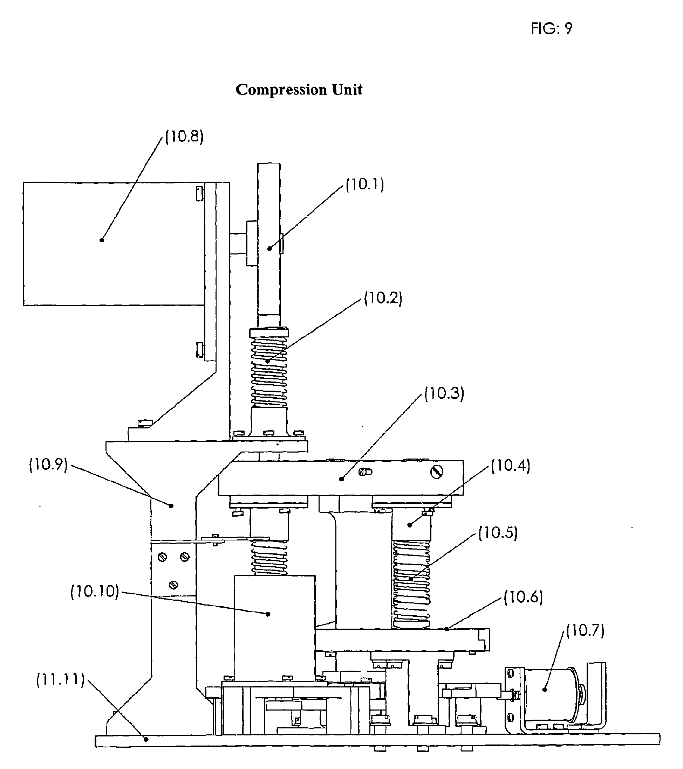

**FIG. 9** is the diagram showing the Compression unit of the invented machinery.

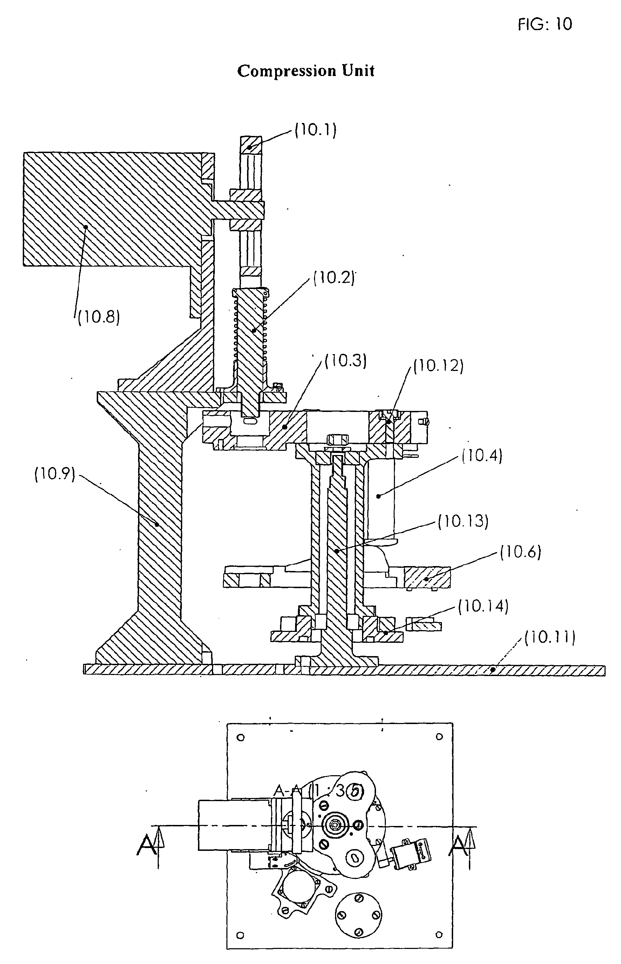

**FIG.10** is the diagram showing the Compression unit of the invented machinery.

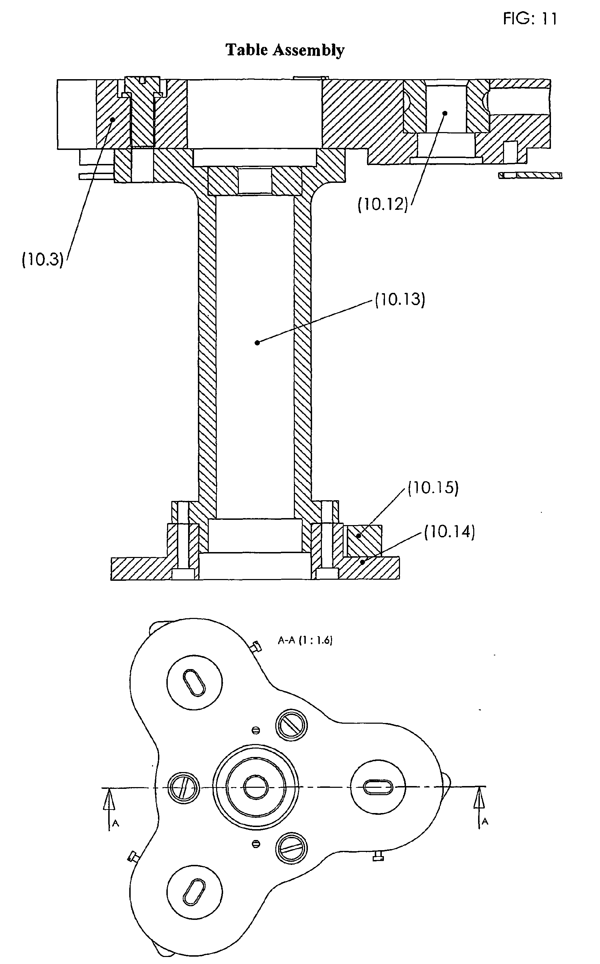

**FIG. 11** is the diagram showing the Table Assembly of the invented machinery.

In the drawings, **1** is the individual ingredient pack with open / close mechanism which can be replaced as and when required. The number of these Individual ingredient packs can be increased or decreased depending upon the requirement. **2** is the measure and draw mechanism which is fully controllable. Sensors aid accurate measurement and drawing of required ingredients from various packs. **3** is the single conveying chute which links the measuring and draw mechanism to the next part.

**4** is the common container in which all the conveying chutes open. **5** is the common interface unit which is basically a conduit tube. **6** is the blender unit consists of different parts. **6.1** is Blender. **6.2** is the Motor 1. **6.3** is the Gear box. **6.4** is the Alignment Chute. **6.5** is the Motor 2. **6.6** is the Motor 3. **6.7** is the Blades inside the Blender. **6.8** is the Support arm. **6.9** is the Bottom Cover. **6.10** is the Motor 4. **6.11** is the Top cover. **7** is the controllable engagement mechanism which is used to attach / detach from the blending unit to the other above part.

**8** is the controllable engagement mechanism which is used to attach / detach from the blending unit to the other below part. **8.1** is the Cam. **8.2** is the Bin Support. **8.3** is the Bracket. **9** is the dosage measuring unit. **9.4** is the Chute. **9.5** is the Motor 1. **9.6** is the Bin. **9.7** is the Black Assembly. **9.8** is the Motor 2. **9.9** is the Motor 3. **9.10** is the Cam. **9.11** is the Sluice Mechanism. **9.12** is the Air Cleaner. **9.13** is the Out Let. **10** is dosage conversion unit which converts the received material into a particular form like tablets, capsules or sachets. **11** is the Compression Unit. **11.1** is the Cam. **11.2** is the Punch Top. **11.3** is the Table Top. **11.4** is the Punch Bottom. **11.5** is the Spring. **11.6** is the Ejector. **11.7** is the Brake. **11.8** is the Motor 1. **11.9** is the Support. **11.10** is the Motor 2. **11.11** is the Base. **11.12** is the Die. **11.13** is the Shaft Support. **11.14** is the Gear. **11.15** is the Lock Piece.

The method of operation is as follows. Each individual ingredient pack(1) is filled with the particular Chemical compound or briefly medicine to the requirement of formulation. Depending upon the data received from the rule engine of the computer, sensors or measure and draw mechanism / Rotary mechanism(2) aid accurate measurement and draw required ingredients from various packs. All such measured and drawn ingredients are delivered into as common container(4) through independent conveying chutes(3).

The common container(4), which receives the ingredients from the conveying chutes(3), delivers the ingredient directly into blender(6) through a common interface unit(5).

This common interface unit(5) is basically a conduit tube connecting the common container(4) and the blender(6), which will obtain the required amount of each of the ingredient as determined by the computer.

Blender(6) that has comprehensive computer controlled mixing mechanism to provide various cycles. Blender contains inner Blades(6.7) for proper mixing or blending the ingredients. The various cycles of rotation for proper blending is controlled by the computer programe through the motor(6.2). Before the cylcles of rotation starts the Top Cover(6.11) and the Bottom Cover(6.9) are closed which are controlled by the computer programe through the Motor(6.6) & Motor(6.10) and Gear Box(6.3). It can control various parameters like number of cycles, angles of rotation, speed of rotation and a proper blended mixture is formed at the end of this process.

Dosage measuring unit(9) receives the mixture from the blender through a controllable engagement mechanism(8), which is fixed at the top of Chute(9.4) of Dosage measuring unit(9). The mixture enters the Bin(9.6) of Dosage measuring unit(9) through the connecting Chute(9.4).

Here the dosage-measuring unit(9) selects the homogeneous mixture depending upon the requirements given by the computer and sends the parted mixtures to the dosage conversion unit(10).

The dosage conversion unit(10) converts the received required part of the mixture in a form of tablet or capsule or sachet or any other form required.

After this particular instant preparation is over the machine is vacuum cleaned through the cleaning system.

Thus the required medicine is manufactured instantly to suit the customised dosage along with the computer printed data having the details like date, Name of Person, Product name, composition, dosage per day, duration for consumption and other information.

## Claims

1. Machinery for manufacturing a batch of dosage of pharmaceutical formulations required for individuals based upon the age, sex, disease, weight, lifestyle etc., comprising:
- differents hoppers (1) on the top;
- a mixer/blender (6) ;
- a dosage-measuring unit (9) ;
- a conversion unit (10),
wherein the blender (6) blends the mixture into a homogeneous mixture, said blender (6) comprising a comprehensive computer controlled mixing mechanism to provide various blending cycles, and **characterized in that**
- various parameters like number of cycles, angles of rotation, speed of rotation can be controlled;
- blending cycle programs are generated in advance, based on certain criteria;
- the mixing concept of the Blending Unit (6) is by revolving a double conical container about a symmetrical radial axis, this unit containing a fixed internal changeable Kneading Unit that effectively and uniformly mixes the ingredients as the container revolves about the axis and also having a controllable opening and closing mechanism (7, 8) at both apex ends of the conical sections, said mechanism (7, 8) allowing free entry for ingredients while drawing them into the Blending Unit (6) and closing the unit while under operation, said apex conical end being adapted for easy engagement/disengagement with the adjacent units;
- after the Measure And Draw Mechanism (2) operation is completed, the top conical end is disengaged with the common Interface Unit (5) and closed, these operations ensuring that the Blending Unit (6) is independent and isolated from other units and is ready for its operation;
- said blender (6) operates as per predetermined blending cycle; and
- once the mixture cycle is completed and the Blending Unit (6) has stopped in the predetermined position, the controllable Engagement Mechanism (8) at the bottom end engages with the receiving chute of the Mixture Storage Unit, the bottom end of the conical section opening up allowing the homogeneously mixed ingredients to flow past it after this engagement.

2. Machinery as defined in claim 1, wherein the dosage Measuring Unit (9) has a controllable Engagement Mechanism (8) at the top of a chute attached to the cover of this unit, the bottom of the chute also having a mechanism used during the cleaning operations.

3. Machinery as defined in claim 1 or claim 2, wherein the dosage Measuring Unit (9) is one of the main units in the whole assembly and is critical in nature, the volumetric measuring working on the principle of a square piston and a square box, where the position of the square piston determines the volume to be measured, the volumetric space being the space enclosed by the frame on three sides, square piston on one side, one sluice gate at the mixture entry side and one sluice gate at the delivery side, a computer controlling the operations of this sluice gate, and the positioning of the piston being pre calibrated to achieve the required volumetric space and being unique for the given mixture of ingredients.

4. Machinery as defined in any of the preceeding claims, wherein the compression Unit (11) consists of a rotary table with indexing facility to stop, fill, punch and eject, the dosage transfer taking place when the table is at halt position, wherein the material does not spill and hence no there is no wastage, and a tablet is formed by compression using a rotating a cam mechanism (11.1).

5. Machinery as defined in any of the preceeding claims, wherein Material-handling units like Chutes (3), Blender (6), Dosage feeding Mechanism (9) and Conversion Unit (10) are cleaned automatically by vacuum cum pressure method, the particulate matter being collected in a disposable bin.

6. An instant process for manufacturing a batch of dosage of pharmaceutical formulations required for individuals based upon the age, sex, disease, weight, lifestyle etc., in an instant process, using an machinery as defined in any of the preceeding claims, wherein different inputs of ingredients are put from the different hoppers (1) on the top, and the ingredients are measured and drawn (2) according to the ratio required, the whole content being sent down through a tube (5), which passes through the mixer/blender (6), to the dosage-measuring unit (9) and to the dosage conversion unit (10) where the whole mixture, depending on the dosage and size, is formed into tablets/capsules/sachets involving several phases of process in single machine.

7. The improved process of claim 6, wherein the hoppers (1) are changed depending on the requirement.

8. The improved process of claim 6, wherein the end formation of the products depends on the form chosen to the need and convenient.

## Patentansprüche

1. Maschine zum Herstellen einer Dosierungscharge pharmazeutischer Formulierungen, die für Einzelpersonen erforderlich sind, auf Grundlage des Alters, des Geschlechts, der Erkrankung, des Gewichts, der Lebensweise usw., wobei die Vorrichtung Folgendes umfasst:
- verschiedene Trichter (1) auf dem Kopfende;
- einen Mischer / Vermischer (6);
- eine Dosierungsmesseinheit (9);
- eine Umrüsteinheit (10),
bei der der Vermischer (6) die Mischung zu einer homogenen Mischung vermischt, wobei der Vermischer (6) einen umfassenden rechnergesteuerten Mischmechanismus umfasst, um verschiedene Vermischungszyklen bereitzustellen, und **dadurch gekennzeichnet, dass**:
- verschiedene Parameter, wie die Zyklenanzahl, die Drehwinkel, die Drehgeschwindigkeit, geregelt werden können;
- Vermischungszyklusprogramme im Vorhinein auf Grundlage bestimmter Kriterien erstellt werden;
- das Mischkonzept der Vermischungseinheit (6) darin besteht, einen doppelkonischen Behälter um eine symmetrische radiale Achse zu drehen, wobei diese Einheit eine feststehende, innere, veränderbare Kneteinheit enthält, die die Bestandteile wirksam und gleichmäßig mischt, während der Behälter sich um die Achse dreht, und außerdem einen regelbaren Öffnungs- und Schließmechanismus (7, 8) an beiden Scheitelenden der konischen Abschnitte aufweist, wobei der besagte Mechanismus (7, 8) einen freien Einlass für Bestandteile ermöglicht, während sie in die Vermischungseinheit (6) gezogen werden und die Einheit im Betrieb geschlossen wird, wobei das besagte konische Scheitelende zur einfachen Einrückung / Ausrückung in die bzw. aus den angrenzenden Einheiten adaptiert ist;
- nachdem der Betrieb des Mess- und Ziehmechanismus (2) beendet wurde, das obere konische Ende aus der gemeinsamen Kopplungseinheit (5) ausgerückt und geschlossen wird, wobei diese Vorgänge sicherstellen, dass die Vermischungseinheit (6) unabhängig ist und von anderen Einheiten getrennt ist und für ihren Betrieb bereit ist;
- der besagte Vermischer (6) gemäß einem vorherbestimmten Vermischungszyklus arbeitet und
- sobald der Mischungszyklus abgeschlossen wurde und die Vermischungseinheit (6) in der vorherbestimmten Stellung angehalten hat, der regelbare Einrückmechanismus (8) am unteren Ende in die Aufnahmerinne der Mischungsaufbewahrungseinheit einrückt, wobei das untere Ende des konischen Abschnitts sich öffnet, um zu ermöglichen, dass die homogen gemischten Bestandteile nach dieser Einrückung an diesem vorbeifließen.

2. Maschine nach Anspruch 1, bei der die Dosierungsmesseinheit (9) einen regelbaren Einrückmechanismus (8) am Kopfende einer Rinne aufweist, die an dem Deckel dieser Einheit angebracht ist, wobei der Boden der Rinne außerdem einen Mechanismus aufweist, der während der Reinigungsvorgänge benutzt wird.

3. Maschine nach Anspruch 1 oder 2, bei der die Dosierungsmesseinheit (9) eine der Haupteinheiten in der Gesamtanordnung ist und wichtig ist, wobei die volumetrische Messung nach dem Prinzip eines quadratischen Kolbens und eines quadratischen Kastens arbeitet, wobei die Stellung des quadratischen Kolbens das zu messende Volumen bestimmt, wobei der Volumenraum der Raum ist, der von dem Rahmen auf drei Seiten, dem quadratischen Kolben auf einer Seite, einem Schleusentor an der Mischungseinlassseite und einem Schleusentor an der Zuführseite umschlossen wird, wobei ein Computer die Arbeitsgänge dieses Schleusentors steuert und die Positionierung des Kolbens vorkalibriert ist, um den erforderlichen Volumenraum zu erzielen, und für die gegebene Mischung von Bestandteilen einzigartig ist.

4. Maschine nach einem der vorhergehenden Ansprüche, bei der die Kompressionseinheit (11) aus einem Drehtisch mit Indexierungseinrichtung zum Anhalten, Befüllen, Stanzen und Auswerfen besteht, wobei der Dosierungstransfer stattfindet, wenn der Tisch sich in der Haltestellung befindet, wobei das Material nicht vergossen wird und somit kein Verlust anfällt und eine Tablette mittels Kompression unter Verwendung eines rotierenden Kurvengetriebes (11.1) geformt wird.

5. Maschine nach einem der vorhergehenden Ansprüche, bei der Materialhandhabungseinheiten wie Rinnen (3), Vermischer (6), Dosierungseinspeisemechanismus (9) und Umrüsteinheit (10) automatisch mittels eines Vakuumdruckverfahrens gereinigt werden, wobei das teilchenförmige Material in einem Einwegbehälter gesammelt wird.

6. Sofortverfahren zum Herstellen einer Dosierungscharge pharmazeutischer Formulierungen, die für Einzelpersonen erforderlich sind, auf Grundlage des Alters, des Geschlechts, der Erkrankung, des Gewichts, der Lebensweise usw., in einem Sofortverfahren, wobei eine Maschine nach einem der vorhergehenden Ansprüche verwendet wird, bei der unterschiedliche Einspeisungen von Bestandteilen aus den verschiedenen Trichtern (1) auf dem Kopfende eingegeben werden und die Bestandteile gemäß dem erforderlichen Verhältnis gemessen und gezogen (2) werden, wobei der gesamte Inhalt durch ein Rohr (5) hinunterbefördert wird und den Mischer / Vermischer (6) zu der Dosierungsmesseinheit (9) und zu der Dosierungsumrüsteinheit (10) durchläuft, wo die gesamte Mischung je nach der Dosierung und Größe zu Tabletten / Kapseln / Briefchen geformt wird, was mehrere Phasen des Verfahrens in einer einzigen Maschine beinhaltet.

7. Verbessertes Verfahren nach Anspruch 6, bei dem die Trichter (1) je nach dem Erfordernis geändert werden.

8. Verbessertes Verfahren nach Anspruch 6, bei dem die Endformung der Produkte von der Form abhängt, die nach dem Bedarf und der Zweckmäßigkeit gewählt wird.

## Revendications

1. - Appareil pour la fabrication d'un lot de dosage de formulations pharmaceutiques requises pour des individus sur la base de l'âge, du sexe, de la maladie, du poids, du style de vie, etc., comprenant :
- différentes trémies (1) sur la partie supérieure ;
- un malaxeur/mélangeur (6) ;
- une unité de mesure de dosage (9) ;
- une unité de conversion (10),
dans laquelle le mélangeur (6) mélange le mélange en un mélange homogène, ledit mélangeur (6) comprenant un mécanisme complet de mélange commandé par ordinateur, pour fournir divers cycles de mélange, et **caractérisé par le fait que** :
- divers paramètres comme le nombre de cycles, les angles de rotation, la vitesse de rotation peuvent être commandés ;
- des programmes de cycles de mélange sont générés à l'avance, sur la base de certains critères ;
- le concept de mélange de l'Unité de Mélange (6) est par révolution d'un conteneur présentant deux cônes autour d'un axe radial de symétrie, cette unité contenant une Unité de Malaxage pouvant être changée, interne, fixe, laquelle mélange efficacement et uniformément les ingrédients alors que le conteneur tourne autour de l'axe et ayant également un mécanisme commandable d'ouverture et de fermeture (7, 8) aux deux extrémités de sommet des sections coniques, ledit mécanisme (7, 8) permettant une entrée libre pour les ingrédients tout en les tirant dans l'Unité de Mélange (6) et fermant l'unité alors qu'elle est en cours de fonctionnement, ladite extrémité conique de sommet étant adaptée pour un engagement/désengagement aisé avec les unités adjacentes ;
- après que le fonctionnement du Mécanisme de Mesure et de Tirage (2) est terminé, l'extrémité conique supérieure est désengagée de l'unité d'interface commune (5) et fermée, ces opérations assurant que l'Unité de Mélange (6) est indépendante et isolée d'autres unités et est prête à fonctionner ;
- ledit mélangeur (6) fonctionne selon un cycle de mélange prédéterminé ; et
- une fois que le cycle de mélange est terminé et que l'Unité de Mélange (6) s'est arrêtée dans la position prédéterminée, le Mécanisme d'Engagement (8) commandable à l'extrémité inférieure s'engage avec la goulotte de réception de l'Unité de Stockage du Mélange, l'extrémité inférieure de la section conique s'ouvrant, permettant aux ingrédients mélangés de façon homogène de s'écouler à travers elle après cet engagement.

2. - Appareil selon la revendication 1, dans lequel l'Unité de Mesure de dosage (9) a un Mécanisme d'Engagement commandable (8) à la partie supérieure d'une goulotte fixée au couvercle de cette unité, la partie inférieure de la goulotte ayant également un mécanisme utilisé pendant les opérations de nettoyage.

3. - Appareil selon la revendication 1 ou la revendication 2, dans lequel l'Unité de Mesure de dosage (9) est l'une des unités principales dans la totalité de l'ensemble et est critique par nature, la mesure volumétrique fonctionnant sur le principe d'un piston carré et d'une boîte carrée, où la position du piston carré détermine le volume à mesurer, l'espace volumétrique étant l'espace fermé par le cadre sur trois côtés, le piston carré d'un côté, une porte de vanne sur le côté d'entrée du mélange et une porte de vanne sur le côté de distribution, un ordinateur commandant les opérations de cette porte de vanne, et le positionnement du piston étant pré-étalonné pour obtenir l'espace volumétrique requis et étant unique pour le mélange d'ingrédients donné.

4. - Appareil selon l'une quelconque des revendications précédentes, dans lequel l'Unité de compression (11) consiste en une table tournante avec un dispositif d'indexage pour arrêter, remplir, perforer et éjecter, le transfert de dosage ayant lieu lorsque la table est à une position d'arrêt, la matière ne se répandant pas et de ce fait il n'y a aucun gaspillage, et un comprimé est façonné par compression à l'aide de la rotation d'un mécanisme de came (11.1).

5. - Appareil selon l'une quelconque des revendications précédentes, dans lequel des unités de manipulation de matière comme les Goulottes (3), le Mélangeur (6), le Mécanisme d'alimentation de Dosage (9) et l'Unité de Conversion (10) sont nettoyées automatiquement par un procédé de vide sous pression, la matière particulaire étant recueillie dans un réservoir jetable.

6. - Procédé instantané pour fabriquer un lot de dosage de formulations pharmaceutiques nécessaires pour des individus sur la base de l'âge, du sexe, de la maladie, du poids, du style de vie, etc., dans un procédé instantané, utilisant un appareil tel que défini à l'une quelconque des revendications précédentes, dans lequel différentes entrées d'ingrédients sont placées à partir des différentes trémies (1) sur la partie supérieure, et les ingrédients sont mesurés et tirés conformément au rapport requis, la totalité du contenu étant adressé pour descendre à travers un tube (5), lequel passe à travers le mélangeur/malaxeur (6), vers l'unité de mesure de dosage (9) et vers l'unité de conversion de dosage (10) où la totalité du mélange, en fonction du dosage et de la dimension, est façonnée en comprimés/capsules/sachets mettant en jeu plusieurs phases de procédé dans une seule machine.

7. - Procédé perfectionné selon la revendication 6, dans lequel les trémies (1) sont changées en fonction de l'exigence.

8. - Procédé perfectionné selon la revendication 6, dans lequel la formation finale des produits dépend de la forme choisie pour le besoin et la commodité.
